# EUROPEAN PATENT SPECIFICATION

(11) **EP 3 077 787 B1**
(45) Date of publication and mention of the grant of the patent: **17.03.2021**
(21) Application number: 14868179.4
(22) Date of filing: 05.12.2014
(51) Int. Cl.: G01N 1/10, G01N 33/28, G01N 1/20

(54) **METHOD FOR ANALYSIS OF A LIQUID WITH A SAMPLING UNIT FOR A LIQUID SAMPLE ADAPTED TO BE FITTED INTO A SYSTEM WITH TEMPERATURE VARIATIONS**
VERFAHREN ZUR ANALYSE EINER FLÜSSIGKEIT MIT EINER EINHEIT ZUR ENTNAHME EINER FLÜSSIGKEITSPROBE ZUM EINBAU IN EINEM SYSTEM MIT TEMPERATURSCHWANKUNGEN
PROCÉDÉ D'ANALYSE D'UN LIQUIDE AVEC UNE UNITÉ D'ÉCHANTILLONNAGE POUR UN ÉCHANTILLON DE LIQUIDE, CONÇUE POUR ÊTRE MONTÉE DANS UN SYSTÈME AYANT DES VARIATIONS DE TEMPÉRATURE

(30) Priority: 06.12.2013 SE 1351456
(43) Date of publication of application: 12.10.2016
(73) Proprietor: Scania CV AB, 151 87 Södertälje (SE)
(72) Inventor: EURENIUS, Klas, 151 46 Södertälje (SE); ERIKSSON, Henrik, S-141 59 Huddinge (SE)
(74) Representative: Scania CV AB
(86) International application number: PCT/SE2014/051457
(87) International publication number: WO 2015/084250

(56) References cited:
- EP-A2- 2 405 251
- WO-A1-2013/002713
- WO-A2-2011/014635
- CN-U- 201 837 541
- DE-A1- 4 301 174
- FR-A1- 2 656 694
- US-A1- 2009 211 379
- US-B1- 7 617 745
- US-B1- 7 874 221

## Description

### FIELD OF TECHNOLOGY

The invention relates to a method for sampling of a liquid.

### BACKGROUND OF THE INVENTION AND PRIOR ART

Diesel powered motor vehicles are equipped with exhaust purification devices with the objective of reducing emissions of particles and chemicals occurring in the diesel engine's exhausts. There are also different standards and statutory requirements governing allowable exhaust emissions from vehicles. Prior art exhaust purification devices are sensitive to high levels of sulphur in the fuel. A level exceeding around 10 ppm sulphur in the fuel may lead to a poor reduction of emissions in the exhaust purification device. In order to reduce this risk and ensure that the legislative requirements are met, some vehicle manufacturers specify the maximum level of sulphur which the fuel may contain, for example that the level of sulphur must be less than 10 ppm. Fuel with higher sulphur levels are often cheaper and therefore more attractive to use in a vehicle. Such fuel damages vehicles, but it is difficult to determine in retrospect and to prove that the vehicle was fuelled with fuel having a sulphur level exceeding 10 ppm.

Other compounds as well, such as phosphorous compounds, may be damaging to the vehicle and more importantly to the environment. However, it is difficult, also in connection with these compounds, to determine in retrospect and to prove that the vehicle was fuelled with fuel having harmful concentrations of such unwanted chemicals or compounds.

In order to reduce emissions of particles and nitrogen oxides (NO_{X}), exhaust treatment systems are used, which may comprise for example a diesel oxidation catalyst (DOC), a particulate filter and so-called NO_{X}-reducers, for example an EGR (Exhaust Gas Reduction)-system and selective catalytic reduction (SCR), in the exhaust stream from combustion engine, for example in vehicles. The efficiency for such exhaust treatment systems and specifically diesel oxidation catalysts is reduced at the occurrence of compounds containing sulphur. Such sulphur-containing compounds (for example mercaptans, thiols, thiophenes, thioethers, thioesters, disulfides) and for example sulphur-containing aromatic compounds, "poison" or react with the diesel oxidation catalyst and/or other components of the exhaust treatment system and these parts of the system therefore have a reduced efficiency leading to corrosion problems in the engine and increased exhaust emissions. The diesel oxidation catalyst is sensitive to high levels of sulphur and may therefore have a shortened life at excessively high sulphur levels in the fuel. It is therefore important to be able to analyse whether the vehicle has been fuelled for a period of time with fuel having too high a sulphur level.

US-2002/0079236 relates to a sensor to measure the concentration of sulphur compounds in a liquid. The sensor comprises two electrodes, one active electrode, which is in contact with the liquid to be measured, and one reference electrode, which is isolated from the liquid. A voltage is generated between the electrodes depending on the concentration of sulphur compounds in the liquid, which may therefore be determined.

US-2009/0317299 relates to an optical sensor to determine the sulphur level in a fuel. This is done by illuminating the fuel with a suitable wavelength spectrum, detecting the reflected light and then analysing this in order to obtain a detection signal, which specifies the sulphur level.

Both these prior art sensors are deemed to be active since they require some form of power supply in connection with the detection or when the signal processing is carried out. The measurements described in both these published patent applications provide a direct measurement result, i.e. a measurement value that reflects the current sulphur level.

A proposed alternative to an electrically powered sensor is described in WO2013002713 A1 which shows an indication unit with a number of capsules or layers that absorb sulphur in contact with the fuel. The capsules contain liquid with different sulphur levels and the layers have differing uptakes of sulphur. At the analysis of the sulphur level in the respective capsules/layers, it is determined whether the level has increased and exceeds the original levels. If so, this is an indication that sulphur from the fuel has been supplied and that the sulphur level in the fuel has exceeded the predetermined level of the relevant capsule/layer.

Despite prior art solutions, there is a need for easily detecting and/or analysing the occurrence and level of sulphur contaminants and other environmentally hazardous compounds in the fuel. There is also a need to collect information about the maximum level of a chemical compound, e.g. sulphur, in the fuel which the combustion engine has been exposed to, since a fuel with too high a sulphur level may deteriorate the function of the catalyst, which in turn means that the emission requirements for the exhausts may not be met. If the catalyst's function deteriorates so that the emission requirements are not complied with, and if the vehicle has not been fuelled with a fuel having a sulphur level exceeding e.g. 10 ppm, this may in the worst case entail that the vehicle manufacturer must recall and repair a large number of vehicles, which may be very costly. If on the other hand it can be proved that the instructions have not been complied with since fuel with more than e.g. 10 ppm has been used, such a campaign will not be necessary.

### SUMMARY OF THE INVENTION

Despite prior art solutions, there is a need further to develop sampling of a fluid, especially of a fuel, during a time period, which gives an indication, in a simple way, of the level of a substance accumulated over such time period. If the sampling unit is used in for example a vehicle, it is possible to analyse whether the vehicle has been fuelled with fuel exceeding predetermined levels for different chemicals or compounds. There is also a need for a sampling unit which is passive and does not require any maintenance and therefore has a low cost.

The objective of the present invention is thus to provide a method for analysis of a liquid with a sampling unit, which in an easy way is adapted to collect a liquid sample, especially a fuel sample, for further analysis. The sample may then provide an indication as to whether the vehicle has been fuelled with fuel having chemical levels exceeding approved levels for the fuel, for example relating to sulphur level.

This objective is achieved with a method as defined in claim 1.

The present invention uses a sampling unit for a liquid sample, preferably for a fuel intended for a combustion engine, which sampling unit is adapted to be fitted into a system with temperature variations. The system contains or transports a liquid to be analysed. The sampling unit comprises a wall section, which partly surrounds a liquid filled cavity.. The sampling device comprises an opening, through which the liquid in the cavity may flow out of the cavity when the liquid expands at temperature increases, and through which the liquid in the system may flow into the cavity when the liquid in the cavity is compressed at a temperature drop. The opening is equipped with a channel, which contains a specified liquid volume. The channel is arranged to be in contact with the liquid in the system.

With such a sampling unit it is possible to adapt the sampling unit for use at a determined temperature area and to continuously collect a liquid sample from a system during a time period, and subsequently to determine the level of a certain substance which the liquid has contained, as and when needed. The time period may be determined based on need. For example, if the DOC-unit's function has deteriorated, there is a need to analyse what fuel has been fuelled.

The liquid in the sampling unit contains a mixture of liquids which have passed through the sampling unit during the sampling period. The mixture of liquids occurs when the liquid in a liquid filled sampling unit expands and/or is compressed in cycles, wherein a small amount of liquid from the system is allowed to flow into the channel or cavity and out of the channel or cavity. The liquid expands when the liquid's temperature rises or is increased. The compression and/or expansion of the liquid is proportionate to the temperature variation and may be determined experimentally and/or by way of calculations. Only a part of the liquid in the sampling unit is replaced at a time, and the liquid in the sampling unit may be given a replacement time, which is determined based on the temperature variation.

When the sampling unit is used in a fuel system to collect a sample from a fuel, it is possible to obtain an indication, with the help of the invention, of for example the sulphur level that a fuel has contained, during for example a long time period. It is therefore possible, for example in connection with too high sulphur levels in the fuel, to obtain an indication of the reason why an exhaust purification device has been put out of service. In the event fuel with a sulphur level above suitable levels has been used and the exhaust purification device has been put out of service, the user may obtain information that (s)he should use fuel with the prescribed sulphur level in the future. In the event the user did not know about the fuel's high sulphur level, the user may make demands on the fuel supplier, who must specify the correct sulphur level of the fuel.

The above objectives are achieved also by using a sampling unit cluster comprising at least two sampling units according to the above.

Furthermore, the above specified objectives are achieved also using a system exposed to temperature variations between a resting temperature and an operating temperature. The operating temperature may be higher than the resting temperature but may also be lower than the resting temperature. The system comprises a hollow component, which contains or transports a liquid to be analysed. The system comprises a sampling unit according to the above fitted into the hollow component, or a sampling unit cluster as described above fitted into the hollow component.

The objectives specified above are also achieved using a combustion engine and a vehicle comprising the combustion engine with a fuel system having a sampling unit or a sampling unit cluster as described above. As there are also different standards and statutory requirements governing permitted exhaust emissions from vehicles it is of interest both to the vehicle manufacturer and the user of the vehicle that the exhaust purification device of the vehicle functions correctly. The sampling unit used in the invention provides both the vehicle manufacturer and the user with an indication as to whether the fuel powering the vehicle's combustion engine has had too high a level of a substance, chemical or compound, for example the sulphur level.

Through the method according to the present invention the level of a substance in the liquid may be analysed easily, for example the level of sulphur in a fuel.

Other features and advantages of the invention are set out in the example descriptions below.

### BRIEF DESCRIPTION OF THE DRAWINGS

Below is a description, as an example, of preferred embodiments of the invention with reference to the enclosed drawings, in which:
Fig. 1 shows a schematic side view of a vehicle with a sampling unit used in the present invention,
Fig. 2 shows an example of a coupling diagram for a fuel system used in the present invention,
Fig. 3a-3h shows a schematic illustration of the sampling unit's function in the present invention,
Fig. 4 shows a schematic cross section view of the sampling unit cluster used in the present invention

### DETAILED DESCRIPTION

The invention is described below with reference to the sampling unit and the method, which is described generally above.

The sampling unit used in the present invention is intended to collect a liquid sample.

Preferably, the liquid is a fuel intended for a combustion engine, but the liquid may be another liquid which may be used within the process industry. When the sampling unit is used, it is fitted into a component of a system, which is subjected to temperature variations. The sampling unit may also be comprised in a sampling unit cluster, which is fitted into a system subjected to temperature variations. The sampling unit of the sampling unit cluster may thus be used in a fuel system or in another system within the process industry.

The sampling unit comprises a wall section, which partly surrounds a liquid filled cavity and may receive the liquid sample. The sampling unit has a shape and a size which is suitable for the liquid sample in question. For example, the cavity may have a drop shape, a conical shape, a cylinder shape or for example the shape of an elongated cylinder. With an elongated cylinder shape, a small contact surface with the liquid may be obtained in relation to the cavity's volume. An elongated cylinder shape may be advantageous since a cylinder shaped sampling unit is easy to manufacture and arrange in different systems. The sampling system may have a volume of approximately 1-20 cm³, for example 5 cm³, but is not limited to such volume.

The sampling unit also comprises an opening, through which the liquid in the system may flow out of the cavity, and through which the liquid may flow into the cavity. The sampling unit is arranged to be in contact with a liquid in the system, e.g. a fuel, which is to be analysed, as an exchange of liquids between the cavity and the surrounding liquid in the system may occur. The liquid sample may be collected continuously during a time period from the liquid, with which the sampling unit is in contact. The time period is determined based on the need for analysis.

The opening of the sampling unit is equipped with a channel, which contains a specified liquid volume. The channel is preferably adapted as an elongated unit, for example as a pipe, which is also called a buffer pipe. This design is simple to manufacture. The volume of the channel is determined based on a temperature variation. The channel is liquid filled and arranged to be in contact with the sampling unit's opening and the liquid in the system. The channel constitutes a buffer that may prevent frequent turnover of liquid in the cavity. The channel counteracts continuous liquid flow into and out of the cavity when the temperature or pressure in the system is stable. Accordingly, the channel constitutes a buffer regulating the exchange of liquid in the sampling unit.

At a stable temperature in the system, there may be minor temperature variations, which are substantially smaller than the variation between the resting and the operating temperature, for example such small temperature variations that occur in the ambient air or at operational disruptions.

The channel's specified volume is preferably adapted to contain a volume of liquid, corresponding to the liquid's expansion or compression volume at a temperature variation, which is smaller than the temperature variation between a resting temperature and an operating temperature. At these minor temperature variations, the liquid compresses/expands less than the volume representing the channel's volume. For example, when the temperature drops, the liquid in the sampling unit compresses. The channel contains a certain volume of liquid and when the liquid's temperature drops, the liquid is compresses and the liquid level in the channel falls. If the compression obtained through the temperature drop is smaller than the channel's volume, the liquid inside the cavity will not be impacted. After the compression the channel may filled up with a new liquid from the system. When the liquid expands at the temperature increase, the channel is first filled with the liquid from the cavity. If the expansion obtained through the temperature increase is smaller than the channel's volume, the liquid inside the cavity will not be pressed out of the channel. If the channel is filled with liquid, a volume of liquid corresponding to the expansion volume is squeezed out of the channel. When the compression volume or the expansion volume caused by the temperature variation is greater than the channel's volume, the exchange of liquids between the system and the cavity will occur, since the liquid in the cavity is impacted. As only a part of the liquid inside the sampling unit is replaced at a time, one may thus obtain an indication of the liquid's composition during the sampling period and accordingly it is also possible to obtain an indication as to unlawful levels of a substance in the liquid.

The channel may also be used to reduce the exchange in a sampling unit at a given temperature - or pressure variation. The volume of the channel is then determined as for example corresponding to a compression volume or expansion volume at a given temperature variation, e.g. 10°C. When the temperature variation is smaller than 10°C, the liquid in the cavity is not impacted. When the temperature variation is greater than 10°C, the compression/expansion impacts the liquid in the cavity as well.

The invention also relates to the use of a sampling unit cluster, comprising at least two sampling units as described above. The sampling units in the cluster comprise channels with varying volumes and therefore the sampling units have different exchange speeds. Such a cluster contains liquid samples, which have been collected during one and the same time period, but which have been impacted by different exchange speeds. This is advantageous especially if the sampling takes place in different temperature areas with different size temperature variations. For example, if the sampling takes place in a fuel system of a truck that is driven both in warm and cold climates, it may be advantageous to equip the vehicle with sampling units equipped with channels having different volumes and accordingly to obtain sampling units adapted to different temperature variation areas. The sampling units in the cluster are preferably arranged in parallel. In this manner, a simple collection of samples may be obtained.

The temperature variation between a resting temperature and an operating temperature may vary greatly, and may for example be anywhere between 5-40°C if the sampling unit is used in the fuel system of a truck, but is not limited to such variation. The resting temperature may correspond to the average ambient temperature, e.g. when the truck engine is turned off. The operating temperature may be higher than the resting temperature, but may also be lower than the resting temperature.

The sampling may occur at temperature variations in the following manner. The cavity is first filled with a starting fluid, which may be a "pure" liquid, e.g. fuel whose sulphur level is below 10 ppm. At resting temperature, which may be the average temperature for the system and may be adapted to the system, for example approximately 20°C, the liquid in the cavity and the channel of the sampling unit are at a normal position, i.e. the cavity and the channel are liquid filled. At an operating state, for example at operation of a truck, the liquid's temperature may increase to the operating temperature inside the cavity so that the liquid expands and a small volume of liquid is squeezed out of the channel. When the system is shut off, the temperature drops again to the resting temperature and the liquid is compressed. Accordingly, the liquid level falls in the channel and the cavity and a small amount of liquid may flow into the cavity and the channel from the system until a normal state with a liquid filled cavity and channel is achieved. During the sampling period several cycles of temperature increases and temperature drops occur.

By sampling in a system with temperature variations as described above, the liquid inside the cavity may be mixed with the liquid in the system a little at a time. The sampling device then obtains a "liquid memory", which is an average composition of the liquids during a time period where several cycles of temperature increases and subsequent temperature drops occur. The exchange time of the liquid inside the cavity varies depending on the temperature variations in the system and the design of the sampling unit and the channel. The exchange times may be produced experimentally or by way of different calculation models. As the exchange of the liquid in the sampling unit occurs relatively slowly, it is possible to check e.g. the average sulphur level over a longer period with the sampling unit. After the sampling period, the contents of the sampling unit are analysed with a suitable method according to prior art.

The sampling unit is especially suitable for collecting fuel samples, especially diesel samples, in a fuel system of a vehicle. The molecules in diesel comprise mainly hydrocarbons between 10 and 22 carbon atoms, e.g. alkanes, aromatics, naphthenes and olefins, but the fuel also contains sulphur compounds and other non-organic compounds such as phosphorous compounds.

The invention also relates to the use of a system, which is subjected to temperature variations between a resting temperature and an operating temperature. The system comprises a hollow component, which contains or transports a liquid. A sampling unit or a sampling unit cluster may be placed or fitted into the hollow component, which may, for example, be a fuel tank.

When the sampling unit or sampling unit cluster is fitted into the component, which may be done, for example, through the wall of the component, the sampling unit or the sampling unit cluster may come into contact with the liquid in the hollowness. The fitting may be carried out in a variety of ways. For example, the wall may be drilled so that a through hole is formed in the wall. Subsequently, the sampling unit or the sampling unit cluster is fitted and fastened in the holes with a suitable attaching device. The sampling unit or the sampling unit cluster may also be shaped as a screw with a hollowness constituting the cavity, and the attachment may be carried out with the help of threads on the outside of the screw. The sampling unit or the sampling unit cluster may be fitted into the component with the help of other attachment methods, and may for example be placed inside a component and screwed onto the inside of the component wall, so that it may come into contact with the surrounding liquid.

The sampling unit or sampling unit cluster is preferably detachably fitted into the hollow component. In this manner, the liquid may be emptied from the cavity easily.

Preferably the system is a fuel system comprising a fuel tank, a fuel conduit, a feeding pump, a fuel filter, a high pressure pump, an accumulator and an injection system. The high pressure pump, the accumulator and the injection system constitute components in the fuel system's high pressure system and the feeding pump and the fuel filter constitute components in the fuel system's low pressure system. The pressure in the high pressure system may be approximately 1800-2500 bar and the pressure in the low pressure system may be approximately 8-15 bar.

At least one sampling unit or sampling unit cluster described above may be fitted into the fuel tank. In this manner, the sampling unit or sampling unit cluster comes into contact with the fuel that has been loaded. It is easy to replace and monitor the sampling unit or sampling unit cluster if it is fitted into the fuel tank.

The sampling unit and/or sampling unit cluster may be fitted into a component in the fuel system's low pressure system. It is easy to replace and monitor the sampling unit or sampling unit cluster if it is fitted into the fuel system's low pressure system. The sampling unit or sampling unit cluster may for example be fitted into the main fuel filter.

The invention also relates to the use of a vehicle comprising the above described fuel system. Preferably the liquid is a fuel and the system is a fuel system and the substance to be analysed is sulphur.

In order to determine the sulphur level of the fuel the liquid must be analysed. This suitably occurs after the sampling unit has been removed from the fuel system, e.g. the fuel system. The sulphur level of the fuel may be analysed according to standard methods such as those described in Swedish Standard SS-EN ISO 20884 (Petroleum products - Determination of sulphur content in fuels - Wavelength-dispersive X-ray spectroscopy (ISO 20884:2011) and / or Swedish Standard SS-EN ISO 20846 (Petroleum products - Determination of sulphur content in fuels - Ultraviolet Fluorescence Method (ISO 20846:2011). From the analysis result an indication is obtained as to whether the tested fuel has a sulphur level that is higher than the recommended level for the fuel, which is below 10 ppm.

Other advantages of the invention are set out in the description below with reference to the enclosed drawings.

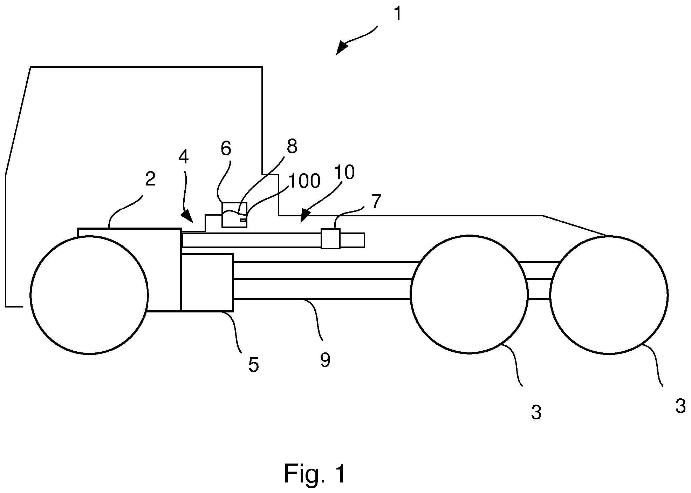

Figure 1 shows a vehicle 1 in a schematic side view, which vehicle 1 is equipped with a combustion engine 2, which operates the driving wheels 3 of the vehicle 1 via a gearbox 5 and a propeller shaft 9. The combustion engine 2 is equipped with an exhaust system 10. The combustion engine 2 is operated by a fuel 8, which is fed to the combustion engine 2 with a fuel system 4 comprising a fuel tank 6. The fuel system 4 also comprises a sampling unit 100, which in this example is placed, in the fuel tank 100. The vehicle also comprises a chassis 7. The exhaust system 10 may comprise for example a diesel oxidation catalyst (DOC), a particulate filter and an EGR-system and an SCR-system.

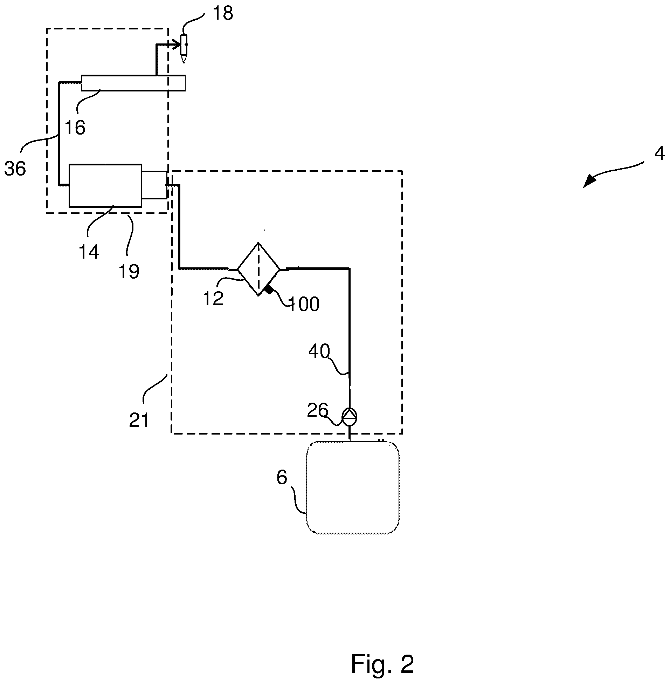

Fig. 2 shows an example of a coupling diagram for a fuel system 4 for a combustion engine 2. The sampling unit 100 may be used for example in such a fuel system, but other versions of the fuel system may be in question. The sampling unit may also be used in other liquid systems, e.g. within the process industry.

The fuel system 4 comprises several components, of which a fuel filter 12, a high pressure pump 14, an accumulator in the form of a so-called common rail 16, and an injection system 18 schematically displayed in the form of fuel injectors arranged in the combustion engine 2 (the combustion engine 2 is displayed in Fig. 1). Alternatively, the common rail 16 may be replaced by another form of an injection system 18, e.g. a piezo- or a unit injection system. The high pressure pump 14, the common rail 16 and the injection system 18 constitute components in the high pressure system 19 of the fuel system 4. A sampling unit or a sampling unit cluster may, according to the present invention, be placed in any of the high pressure system's components, for example in a fuel conduit between the high pressure pump 14 and the common rail 16.

The fuel system 4 also comprises a fuel tank 6 and a feeding pump 26. These components may be arranged at the vehicle's chassis 7 (chassis 7 is displayed in Fig. 1). The fuel filter 12 is arranged downstream of the pump 26 and upstream of the high pressure pump 14 in the fuel system 4. The sampling unit 100 may be fitted into the fuel filter 12, as displayed in this example, but other placements are also possible, for example in a fuel conduit 40.

The feeding pump 26 pressurises the fuel in a low pressure system 21 of the fuel system and feeds the fuel from the fuel tank 6 via the fuel conduit 40 through the fuel filter 12 and further along to the high pressure pump 14. The fuel is then fed, at a high pressure, to the common rail 16 and further along to the injection system 18.

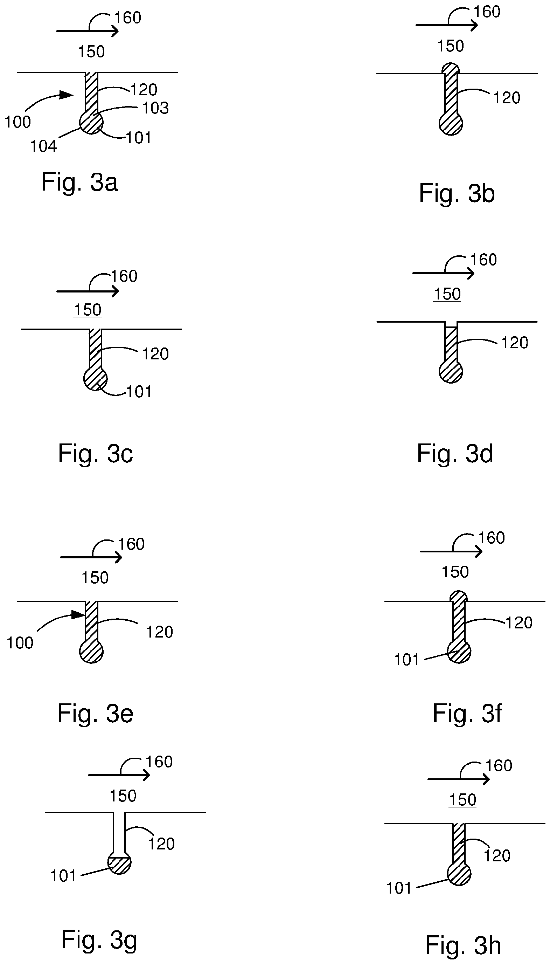

Fig. 3a-3h schematically illustrate the function of the sampling unit 100 used in the present invention. Fig. 3a shows a sampling unit 100, which comprises a wall section 104 that partly surrounds a cavity 101, an opening 103 and a channel in the form of a buffer pipe 120 between a component 150 and the opening 103 of the sampling unit 100. The liquid inside the component 150 flows in the direction of the arrow 160. The pipe 120 is a channel, which is liquid filled and has a volume that can hold a volume of liquid, corresponding to the liquid's expansion or compression volume at a temperature variation, which is smaller than the temperature variation between a resting temperature and an operating temperature. Accordingly the pipe 120 may receive or emit a small amount of liquid when only a small temperature variation or pressure variation occurs in the system. The parts of the sampling unit are identical in all the drawings 3a-3h, but to increase the clarity in the drawings, all parts are shown only in Fig. 3a.

Fig. 3a illustrates the sampling unit at a resting temperature. The pipe 120 is filled with a liquid. Fig. 3b shows how the liquid expands when a temperature increase takes place and some of the liquid inside the buffer pipe 120 is squeezed out of the buffer pipe 120 in the component 150. Fig. 3c illustrates the situation after the liquid, which has been squeezed out of the pipe 120, has been washed away because of the movements of the surrounding liquid in the component 150. The temperature in the system is stable and the sampling unit is in a normal position in Fig. 3c, i.e. both the cavity 101 and the buffer pipe 120 are liquid filled. Fig. 3d shows that the liquid is compressed when the temperature of the liquid drops and the liquid level in the channel 120 drops. New liquid may therefore flow into the pipe 120 from the component 150 and a new normal position, as shown in Fig. 3a, may be achieved. The temperature variation occurring in the system, and which is displayed in Fig. 3a-3d is smaller than the variation between the resting temperature and the operating temperature.

Fig. 3e-3h show a situation in which the temperature change occurs between a resting temperature and an operating temperature. Fig. 3e illustrates the sampling unit 100 at a resting temperature. Fig. 3f shows how the liquid expands when a temperature increase from the resting temperature to the operating temperature occurs. At expansion, the liquid expands in the buffer pipe 120 and in the cavity 101 and a small amount of liquid is squeezed out of the pipe 120. When the temperature drops from the operating temperature to the resting temperature, the liquid is compressed with a volume corresponding to the volume of the entire pipe 120 and with a part of the volume of the cavity 101, as displayed in Fig. 3g. Then, a corresponding amount of liquid from the component 150 may flow into the cavity 101 and the pipe 120 and a new normal position, in which both the cavity and the pipe are liquid filled, is achieved for the sampling unit, as shown in Fig. 3h.

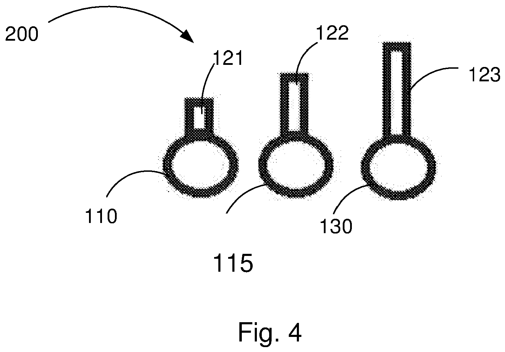

Fig. 4 illustrates a cluster 200 of sampling units 110, 115 and 130. Each one of the sampling units comprises a channel, referred to as 121, 122 and 123. Each channel has a varying length and a varying volume, facilitating adaptation of the sampling units to a desired temperature variation interval.

The foregoing description of the preferred embodiments of the present invention has been furnished for illustrative and descriptive purposes. The described embodiments are not intended to be exhaustive or to limit the invention, but the invention is limited by the scope of the enclosed claims.

## Claims

1. Method for analysis of a liquid with a sampling unit (100) or with a sampling unit cluster (200) comprising at least two sampling units (110, 115, 130), which sampling unit (100) and sampling unit cluster (200) are adapted to be fitted into a system with temperature variations, which system contains or transports a liquid to be analysed, wherein the sampling unit (100, 115, 120, 130) comprises a wall section (104) partly surrounding a cavity (101) for being filled with a liquid, and an opening (103) through which the liquid in the cavity may flow out of the cavity (101) when the liquid expands at a temperature increase, and through which opening (103) the liquid in the system may flow into the cavity (101) when the liquid in the cavity is compressed at a temperature drop, which opening (103) is equipped with a channel (120, 121, 122, 123) that may hold a specified volume of liquid and which channel (120, 121, 122, 123) is arranged to be in contact with the liquid in the system, wherein the method comprises the steps:
a) of filling the cavity (101) in the sampling unit (100, 115, 120, 130) with a starting liquid;
b) of placing or fitting the sampling unit (100) or the sampling unit cluster (200) in the system, in which the sampling unit (100, 115, 120, 130) comes into contact with the liquid to be analysed;
c) of subjecting the system to temperature variations between a resting temperature and an operating temperature and vice versa;
d) of allowing the liquid in the channel (120, 121, 122, 123) to flow out of the channel (120, 121, 122, 123) and into the channel (120, 121, 122, 123) at a temperature variation, which is smaller than the temperature variation between the resting temperature and the operating temperature;
e) of allowing the liquid both in the channel (120, 121, 122, 123) and in the cavity (101) to flow out of the channel (120, 121, 122, 123) and the cavity (101) and into the channel (120, 121, 122, 123) and the cavity (101) at a temperature variation between the resting temperature and the operating temperature;
f) of allowing mixture of the liquid inside the cavity (101) by way of the liquid's in- and out-flow;
g) of removing the sampling unit (100) or the sampling unit cluster (200) from the system after a time period,
h) of removing the liquid accumulated in the sampling unit (100) or in the sampling unit cluster (200), and
i) of analysing the level of a substance in the liquid from the sampling (100) or the sampling unit cluster (200) with an analysis method adapted to the substance to be analysed.

2. Method according to claim 1, **characterised in that** the liquid is a fuel and the system is a fuel system (4).

3. Method according to claim 2, **characterised in that** the substance to be analysed is sulphur.

## Patentansprüche

1. Verfahren zur Analyse einer Flüssigkeit mit einer Probenahmeeinheit (100) oder mit einem Probenahmeeinheit-Cluster (200), der mindestens zwei Probenahmeeinheiten (110, 115, 130) umfasst, wobei die Probenahmeeinheit (100) und der Probenahmeeinheit-Cluster (200) dazu eingerichtet sind, in ein System mit Temperaturschwankungen eingesetzt zu werden, wobei das System eine zu analysierende Flüssigkeit enthält oder transportiert, wobei die Probenahmeeinheit (100, 115, 120, 130) einen Wandabschnitt (104), der eine mit einer Flüssigkeit zu befüllende Vertiefung (101) teilweise umgibt, und eine Öffnung (103) aufweist, durch die die Flüssigkeit in der Vertiefung aus der Vertiefung (101) herausfließen kann, wenn sich die Flüssigkeit bei einer Temperaturzunahme ausdehnt, und durch welche Öffnung (103) die Flüssigkeit in dem System in die Vertiefung (101) hinein fließen kann, wenn die Flüssigkeit in der Vertiefung sich bei einer Temperaturabnahme zusammenzieht, wobei die Öffnung (103) mit einem Kanal (120, 121, 122, 123) versehen ist, der ein festgelegtes Flüssigkeitsvolumen enthalten kann, und wobei der Kanal (120, 121, 122, 123) dazu angeordnet ist, in Verbindung mit der Flüssigkeit in dem System zu stehen,
wobei das Verfahren die Schritte umfasst:
a) Füllen der Vertiefung (101) in der Probenahmeeinheit (100, 115, 120, 130) mit einer Startflüssigkeit,
b) Platzieren oder Einsetzen der Probenahmeeinheit (100) oder des Probenahmeeinheit-Clusters (200) in das System, in dem die Probenahmeeinheit (100, 115, 120, 130) in Kontakt mit der zu analysierenden Flüssigkeit kommt,
c) Aussetzen des Systems gegenüber Temperaturänderungen zwischen einer Ruhetemperatur und einer Betriebstemperatur und umgekehrt,
d) Gestatten der Flüssigkeit in dem Kanal (120, 121, 122, 123), bei einer Temperaturänderung, die kleiner ist als die Temperaturänderung zwischen der Ruhetemperatur und der Betriebstemperatur, aus dem Kanal (120, 121, 122, 123) heraus und in den Kanal (120, 121, 122, 123) hinein zu strömen,
e) Gestatten der Flüssigkeit sowohl in dem Kanal (120, 121, 122, 123) und in der Vertiefung (101), bei einer Temperaturänderung zwischen der Ruhetemperatur und der Betriebstemperatur aus dem Kanal (120, 121, 122, 123) und der Vertiefung (101) heraus und in den Kanal (120, 121, 122, 123) und die Vertiefung (101) hinein zu strömen,
f) Zulassen einer Vermischung der Flüssigkeit innerhalb der Vertiefung (102) durch das Hinein- und Herausströmen der Flüssigkeit,
g) Entnehmen der Probenahmeeinheit (100) oder des Probenahmeeinheit-Clusters (200) aus dem System nach einer Zeitdauer,
h) Entnehmen der in der Probenahmeeinheit (100) oder in dem Probenahmeeinheit-Cluster (200) angesammelten Flüssigkeit, und
i) Analysieren des Gehalts einer Substanz in der Flüssigkeit aus der Probenahmeeinheit (100) oder dem Probenahmeeinheit-Cluster (200) mit einem auf die zu analysierende Substanz abgestimmten Analyseverfahren.

2. Verfahren nach Anspruch 1, **dadurch gekennzeichnet, dass** die Flüssigkeit ein Kraftstoff ist und das System ein Kraftstoffversorgungssystem (4) ist.

3. Verfahren nach Anspruch 2, **dadurch gekennzeichnet, dass** die zu analysierende Substanz Schwefel ist.

## Revendications

1. Procédé pour une analyse d'un liquide avec une unité d'échantillonnage (100) ou avec un groupe d'unités d'échantillonnage (200) comprenant au moins deux unités d'échantillonnage (110, 115, 130), lesquels unité d'échantillonnage (100) et groupe d'unités d'échantillonnage (200) sont adaptés pour être ajustés dans un système avec des variations de température, lequel système contient ou transporte un liquide à analyser, dans lequel l'unité d'échantillonnage (100, 115, 120, 130) comprend une section de paroi (104) entourant partiellement une cavité (101) à remplir avec un liquide, et une ouverture (103) à travers laquelle le liquide dans la cavité peut s'écouler hors de la cavité (101) lorsque le liquide se dilate lors d'une augmentation de température, et à travers laquelle ouverture (103) le liquide dans le système peut s'écouler dans la cavité (101) lorsque le liquide dans la cavité est comprimé lors d'une baisse de température, laquelle ouverture (103) est équipée d'un canal (120, 121, 122, 123) qui peut contenir un volume spécifié de liquide et lequel canal (120, 121, 122, 123) est agencé pour être en contact avec le liquide dans le système,
dans lequel le procédé comprend les étapes :
a) de remplissage de la cavité (101) dans l'unité d'échantillonnage (100, 115, 120, 130) avec un liquide de démarrage ;
b) du placement ou de l'ajustement de l'unité d'échantillonnage (100) ou du groupe d'unités d'échantillonnage (200) dans le système, dans lequel l'unité d'échantillonnage (100, 115, 120, 130) vient en contact avec le liquide à analyser ;
c) de soumission du système à des variations de température entre une température de repos et une température de fonctionnement et vice versa ;
d) de possibilité pour le liquide dans le canal (120, 121, 122, 123) de s'écouler hors du canal (120, 121, 122, 123) et dans le canal (120, 121, 122, 123) à une variation de température, qui est inférieure à la variation de température entre la température de repos et la température de fonctionnement ;
e) de possibilité pour le liquide à la fois dans le canal (120, 121, 122, 123) et dans la cavité (101) de s'écouler hors du canal (120, 121, 122, 123) et de la cavité (101) et dans le canal (120, 121, 122, 123) et la cavité (101) à une variation de température entre la température de repos et la température de fonctionnement ;
f) de possibilité du mélange du liquide à l'intérieur de la cavité (101) au moyen du flux d'entrée et de sortie du liquide ;
g) de retrait de l'unité d'échantillonnage (100) ou du groupe d'unités d'échantillonnage (200) du système après une période de temps,
h) de retrait du liquide accumulé dans l'unité d'échantillonnage (100) ou dans le groupe d'unités d'échantillonnage (200), et
i) d'analyse du niveau d'une substance dans le liquide à partir de l'unité d'échantillonnage (100) ou du groupe d'unités d'échantillonnage (200) avec un procédé d'analyse adapté à la substance à analyser.

2. Procédé selon la revendication 1, **caractérisé en ce que** le liquide est un carburant et le système est un système de carburant (4) .

3. Procédé selon la revendication 2, **caractérisé en ce que** la substance à analyser est du soufre.
